# EUROPEAN PATENT APPLICATION

(11) **EP 4 570 193 A1**
(43) Date of publication of application: **18.06.2025**
(21) Application number: 23851730.4
(22) Date of filing: 04.08.2023
(51) Int. Cl.: A61B 17/04

(54) **IMPLANT AND STAPLER**

(30) Priority: 11.08.2022 CN 202210964132
(71) Applicant: Hangzhou Rejoin Mastin Medical Device Co., Ltd., Hangzhou, Zhejiang 311100 (CN)
(72) Inventor: HU, Jiancheng, Hangzhou, Zhejiang 311100 (CN); LI, Bin, Hangzhou, Zhejiang 311100 (CN); JIANG, Guansen, Hangzhou, Zhejiang 311100 (CN); XU, Shengjun, Hangzhou, Zhejiang 311100 (CN)
(74) Representative: von Tietzen und Hennig, Nikolaus
(86) International application number: PCT/CN2023/111292
(87) International publication number: WO 2024/032509

(57) **Abstract**

The embodiments of the present disclosure provide an implant and a suturing device. A first chamfer is provided between a front end surface of the implant and a circumferential sidewall of the implant. A second chamfer is provided between a rear end surface of the implant and the circumferential sidewall of the implant. The suturing device comprises a puncture needle and one or more implants. The one or more implants are located on the puncture needle. The puncture needle is provided with a channel extending in an axial direction. The one or more implants can be triggered from the suturing device through the channel . At least two limiting structures are provided on an inner wall of the channel . The at least two limiting structures are arranged along a circumferential direction; and the at least two limiting structures can at least block a second implant to be triggered, such that an applied force is increased when the second implant to be triggered is triggered out of the suturing device.

## Description

### CROSS-REFERENCE TO RELATED APPLICATIONS

This disclosure claims priority to the Chinese patent application entitled "Implant and Suturing Device" (Application No. 202210964132.0), which was filed with the China National Intellectual Property Administration on August 11, 2022. The entire contents of which are incorporated herein by reference..

### TECHNICAL FIELD

The present disclosure relates to the technical field of medical devices, and in particular to an implant and a suturing device.

### BACKGROUND

When suturing tissues with a suturing device, a puncture needle may encounter an obstacle during puncturing, causing the puncture needle to bend slightly, and an implant disposed on the puncture needle may squeeze the implant upon contact with a sidewall of the bent puncture needle to cause the implant to break. Therefore, it is desirable to provide an implant and a suturing device to alleviate the problem of breakage of the implant caused by the existing implant being squeezed after the puncture needle is bent.

### SUMMARY

The present disclosure provides an implant with a first chamfer between a front end face of said implant and a circumferential side wall of said implant; and a second chamfer between a rear end face of said implant and a circumferential side wall of said implant. The first chamfer and the second chamfer can serve to avoid the inner wall of the puncture needle that is bent after the puncture needle has been bent, avoiding the puncture needle to immediately butt against the end of the implant after a small bend has been made, which can lead to fracture of the implant.

In some embodiments, said bottom surface of the implant is planar. The bottom surface of the implant can form a gap with the inner wall of the circular hole inside the puncture needle, which can avoid the inner wall of the puncture needle immediately contacting the implant after bending, leading to fracture of the implant.

In some embodiments, said first chamfer and second chamfer may take values in the range of 8-10°. The larger the angle of the first and second chamfers the better the avoidance effect, but the angle if too large the strength of the implant is reduced.

The present disclosure provides a suture apparatus comprising: a puncture needle and a plurality of said implants, said implants being disposed on said puncture needle; said puncture needle being provided with a channel extending in the axial direction, the plurality of said implants being able to be struck from within said suture apparatus after passing through said channel in sequence; said channel being provided with a plurality of limiting structures protruding inwardly from the inner wall thereof.

The plurality of limit structures are arranged in a circumferential row, said limit structures being capable of acting as a block to at least a second struck said implant so as to increase the force exerted when the second implant is struck out of said suture apparatus. The limit structures are located on the strike path of the implant, the limit structures are provided in such a way that they are able to act at least as a block to the second implant, the friction on the second implant increases under the blocking effect of the circumferential plurality of limit structures, the second implant is more difficult to pass through the location where the limit structures are provided, and a greater external force needs to be exerted on the second implant in order to get the second implant through the limit structures, which reduces risk of the first implant bringing out the second implant.

In some embodiments, said limit structure comprises a guiding bevel disposed at an angle with respect to said inner wall of said channel, said implant being in sliding contact with said guiding bevel, said guiding bevel being used to guide said implant over said limit structure. Under the action of the guiding bevel, the pressure of the limiting structure on the implant gradually increases, the friction force on the implant gradually increases, and the limiting structure is prevented from completely blocking the second implant.

In some embodiments, said limiting structure is able to act as a block for all said implants. All of the implants, in particular the first implant, are able to be blocked by the limiting structure, thus avoiding the first implant other than the first implant from slipping out of the puncture needle. The shape of all the implants can be identical and the preparation of the implants is easier and more versatile.

In some embodiments, the first said implant is provided with an avoidance structure that avoids said limit structure so that the first said implant is subjected to less friction when passing through said limit structure than the second said implant is when passing through said limit structure, facilitating the striking of the first implant.

In some embodiments, said puncture needle comprises a hollow bore, a notch provided in a circumferential side wall of said puncture needle in communication with said hollow bore; axially, a front end of said notch is in communication with a tip end surface of said puncture needle, said notch and said hollow bore enclosing a slot structure, said slot structure forming said passageway; and said limiting structure comprises a projection disposed on an inner surface of said slot structure. The two groove walls of the groove structure can undergo a small deformation so as to increase the width of the notch opening of the groove structure, and the groove walls drive the projections to press against the side wall of the implant so as to increase the friction, and the groove structure can be set up to conveniently realize the blocking effect on the second implant by using the deformation changes of itself, and the structure is simpler.

In some embodiments, said groove structure is provided with projections on two opposite side walls, and the two projections are provided opposite to each other, so that the implant is subjected to uniform force.

In some embodiments, at least a portion of said implant is disposed within said hollow aperture, and when the portion of said implant disposed within said hollow aperture is pressed against the opening of said slot structure, there is a deformation gap between the bottom surface of said implant and the bottom surface of the slot structure, thereby providing space for bending of the puncture needle.

In some embodiments, said deformation gap ranges from 0.05 mm to 0.4 mm. the larger the value of the gap, the less likely the implant is to be extruded against the puncture needle, but too large a value can result in the implant being too small or the puncture needle being too thick.

In some embodiments, said implant comprises a first portion and a second portion sequentially connected in a top-down direction along the depth of the slot structure; said first portion has a suspension structure on a side wall of said first portion, said first portion being suspended from said notch by said suspension structure so as to provide a gap between the bottom of the second portion and the inner side wall of the hollow aperture; said second portion being disposed within said hollow aperture, and said second portion of said second portion at its widest position is greater than the width of said opening, allowing for a larger deformation space while the implant is suspended from the puncture needle.

In some embodiments, said suture comprises a fixation portion and a striking mechanism, said puncture needle being coupled to said fixation portion, said striking mechanism being capable of axial movement with respect to said fixation portion, said striking mechanism being used to strike said implant toward the anterior end.

In some embodiments, said striking mechanism comprises a push pin, a push button, a connecting arm and a linkage portion, said push button, connecting arm and linkage portion being disposed sequentially from the front end of said suture towards the rear end; said front end of said push pin being disposed in a hollow bore of said puncture needle; said push button being slidably connected to said fixation portion; said front end of said connecting arm being connected to said push button, and the rear end of said connecting arm being connected to the linkage portion; Said linkage portion is connected to the rear end of the push pin. By moving the push pin by the connecting arm and the linkage section, the push button can be set in a position that is convenient for the operator to control, so that the structure of the suture apparatus is more in line with ergonomics.

In some implementations, a spring is provided between said fixing portion and said linkage portion for resetting the push button.

In some embodiments, said suture apparatus further comprises a depth-limiting tube and a locking mechanism, wherein said depth-limiting tube is socketed to the outer side of said puncture needle, wherein the anterior end of said depth-limiting tube is a depth-limiting end; wherein said depth-limiting tube is adjustable in axial direction; and wherein said locking mechanism is used to lock said depth-limiting tube in place so as to fix the position of said depth-limiting tube relative to the puncture needle. Before suturing, the thickness of the tissue to be sutured can be measured, and then the position of the depth-limiting tube can be adjusted and locked so that the length of the portion of the puncture needle exposed at the front end of the depth-limiting tube matches the thickness of the tissue to be sutured, improving the efficiency of the procedure.

In some embodiments, said locking mechanism comprises a control arm, said control arm comprising a first connecting arm, a press arm and a second connecting arm connected in an axial direction in sequence, one end of said first connecting arm being connected to a depth limiting tube and the other end being connected to one end of the press arm, one end of said second connecting arm being connected to the other end of the press arm, and the other end of said second connecting arm being connected to a fixed portion on a sliding basis; said press arm having a gap between said press arm and said fixed portion said locking mechanism further comprises a plurality of limit slots located on the housing, the plurality of limit slots being sequentially arranged in the axial direction; said pressure arm has a locked state and an unlocked state, in the locked state, said gear pin is located in said limit slots, and said limit slots are used to prevent axial movement of said pressure arm, and in the unlocked state, said gear pin is located in said limit slots, and said limit slots are used to prevent axial movement of said pressure arm, and in the unlocked state, said gear pin is located in said limit slots, and in the unlocked state, said gear pin is located in the lock state. In the unlocked state, said gear pin is pressed out of said limit slot and said press arm is able to move in the axial direction. By pressing and sliding the press arm, a change of gear position is realized, which is simple to control.

In some embodiments, said fixed portion is provided with a first guiding groove extending in the axial direction, said first connecting arm is provided with a sliding resisting portion, said sliding resisting portion resists the first guiding groove, said first guiding groove is used for guiding said first connecting arm to slide along the axial direction of said puncture pin, thereby avoiding rotation of the first connecting arm, so that the first connecting arm is always sliding in the axial direction.

In some embodiments, said suture apparatus comprises a housing and a striking mechanism, said striking mechanism being used to strike said implant towards the front end; said housing is provided with a stall groove on the inner wall of said housing; said striking mechanism is provided with an elastic pendulum, a fixed end of said elastic pendulum being fixedly connected to the striking mechanism, and a free end of said elastic pendulum being extended from the groove of said stall groove into said stall groove. The stall slot is provided on the housing, which has a simple structure and saves cost.

In some embodiments, the number of implants within said suture apparatus is two, said stall slot comprising a first slot unit and a second slot unit; along the direction of the rear end towards the front end, said first slot unit comprises a first starting end and a first limiting end disposed at opposite ends thereof; said second slot unit comprises a second starting end and a second limiting end disposed at opposite ends thereof; said stall slot further comprising a first recession slot, said the bottom surface of said first slot unit is lower than the bottom surface of said first slot unit; one end of said first slot is located on the bottom surface of said first limit end and extends from said first limit end towards the rear end and through the side wall of said first slot unit to connect with the second slot unit, and the extension direction of said first slot is at an angle with the extension direction of said first slot unit; the bottom surface of said second slot unit is lower than the bottom surface of said first slot bottom surface of said first slot unit; said stall slot further comprising a second receding slot, the bottom surface of said second receding slot being lower than the bottom surface of said second slot unit; one end of said second receding slot being disposed on the bottom surface of said second limiting end and extending from said second limiting end towards the rear end and being in communication with a first starting end; said free end being disposed at the first starting end of the first slot unit in an initial state and said free end having a tendency to move towards said stall slot's bottom surface with a tendency to move toward the bottom surface of said stall slot so that said free end always resists the bottom surface of said stall slot. The surgeon needs to pay attention to and confirm the state and number of implant strikes during surgery, and the stall slot better limits the number and sequence of implant strikes.

In some embodiments, the number of implants in said suture device is two, said slot comprising a first slot unit and a second slot unit; along the rear end towards the front end, said first slot unit comprises a first starting end and a first limiting end located at opposite ends thereof; said second slot unit comprises a second starting end and a second limiting end located at opposite ends thereof; said first slot unit comprises a first slot wall and a second slot said first slot unit comprises a first slot wall and a second slot wall, said first slot wall extending in a direction parallel to the axial direction, with the width between said second slot wall and said first slot wall gradually decreasing along the rear end towards the front end; said second slot unit is located on the side where the second slot wall of said first slot unit is located; said first slot unit and the second slot unit have a first receding groove between them, with said first receding groove having a first transverse groove at the front end connecting with the first limiting end of the first slot unit. The rear end of said first recession slot is connected to the second start end of said second slot unit by a second horizontal slot; said second slot unit is also provided with a second recession slot on the side of said second slot unit, and said second slot unit is located between said first recession slot and said second recession slot; the front end of said second recession slot is connected to the second limit end by a third horizontal slot, and the rear end of said second recession slot is connected to the wall of the second slot, and the bottom surface of said second recession slot is higher than that of said first slot unit, and said second recession slot is connected to the second slot unit by a third horizontal slot, and said second recession slot is connected to the wall of the second slot by a second horizontal slot. end bottom surface is higher than the bottom surface of the first starting end of said first slot unit, thereby forming a lower stepped structure at the connection between the second recession slot and the first slot unit; in the initial state, said free end is disposed within the first slot unit and is disposed behind and against the second slot wall of the rear end opening of said second recession slot. The surgeon needs to note and confirm the state and number of implant strikes during surgery, and the stall slots better define the number and sequence of implant strikes.

In some embodiments, the material of said housing is transparent, which facilitates the control of the suture apparatus by the operator, and at the same time allows better observation of the puncture process.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1 is a schematic diagram illustrating an implant according to some embodiments of the present disclosure;
FIG. 2 is a schematic diagram illustrating a suturing device according to some embodiments of the present disclosure;
FIG. 3 is a schematic diagram illustrating a partial enlargement of a position A in FIG. 2;
FIG. 4 is a schematic diagram illustrating a cross-section of a puncture needle of a suturing device according to some embodiments of the present disclosure;
FIG. 5 is a schematic diagram illustrating a suturing device excluding an upper half housing according to some embodiments of the present disclosure;
FIG. 6 is a schematic diagram illustrating a front view of a suturing device according to some embodiments of the present disclosure;
FIG. 7 is a schematic diagram illustrating a cross-sectional view in an A-A direction in FIG. 6;
FIG. 8 is a schematic diagram illustrating a cross-sectional view in a B-B direction in FIG. 6;
FIG. 9 is a schematic diagram illustrating a gear slot of a suturing device according to some embodiments of the present disclosure;
FIG. 10 is a schematic diagram illustrating a movement path of a free end in the gear slot in FIG. 9;
FIG. 11 is a schematic diagram illustrating another gear slot of a suturing device according to some embodiments of the present disclosure; and
FIG. 12 is a schematic diagram illustrating a movement path of a free end in the gear slot in FIG. 11.

Reference signs: 100-puncture needle; 110-channel; 120-limiting structure; 121-guide slope; 210-first implant; 220-second implant; 230-first portion; 240-second portion; 310-deformation clearance; 320-first chamfer; 330-second chamfer; 400-fixing portion; 510-push pin; 520-push button; 530-connection arm; 540-linkage portion; 550-spring; 610-depth limiting tube; 621-first connection arm; 622-pressing arm; 6221-gear pin; 623-second connection arm; 630-limiting slot; 640-first guide slot; 650-sliding abutting portion; 660-button; 710-first longitudinal rod; 720-first vertical rod; 730-second longitudinal rod; 740-second vertical rod; 750-limiting buckle; 760-free end; 810-first slot unit; 811-first starting end; 812-first limiting end; 813-second slot wall; 820-first retreat slot; 830-second slot unit; 831-second starting end; 832-second limiting end; 840-first transverse slot; 850-second transverse slot; 860-third transverse slot; 870-second retreat slot; 900-housing; 910-threaded member.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation. It should be understood that the terms "system", "device", "unit" and/or "module" used herein are a way to distinguish between different components, elements, parts, sections, or assemblies at different levels. However, the terms may be replaced by other expressions if other words accomplish the same purpose. As shown in the present disclosure and in the claims, unless the context clearly suggests an exception, the words "one", "a", "an", "one kind", and/or "the" do not refer specifically to the singular, but may also include the plural. Generally, the terms "including" and "comprising" suggest only the inclusion of clearly identified steps and elements, however, the steps and elements that do not constitute an exclusive list, and the method or apparatus may also include other steps or elements.

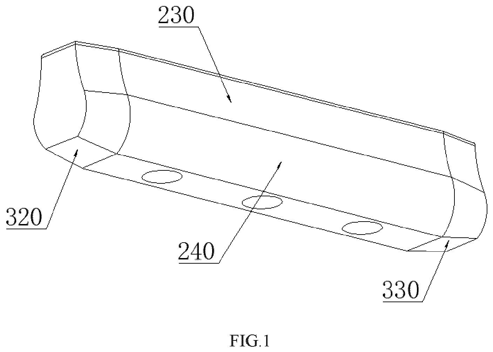

In some embodiments, as shown in FIG. 1, a first chamfer 320 is provided between a front end surface of the implant and a circumferential sidewall of the implant; and a second chamfer 330 is provided between a rear end surface of the implant and the circumferential sidewall of the implant. In some embodiments, a plurality of circumferential sidewalls of the implant are provided with the first chamfer 320, respectively. In some embodiments, the first chamfer 320 is provided in one or more circumferential sidewalls of the implant except an upper end sidewall. An upper end of the implant refers to a side of the implant away from a human body after the implant is placed in a suturing device when the suturing device is used. In some embodiments of the present disclosure, when a puncture needle 100 penetrates into the human body, the puncture needle 100 may bend slightly. The first chamfer 320 and the second chamfer 330 can help avoid an inner wall causing the puncture needle to bend, thereby preventing the puncture needle 100 from immediately contacting an end portion of the implant after the puncture needle bends slightly to cause the implant to break.

In some embodiments, the implant may include a first portion 230 and a second portion 240. When the implant is placed in the puncture needle, the first portion 230 may be located at an upper end of the puncture needle, and the second portion 240 may be located at a lower end of the puncture needle. In some embodiments, the implant may include the first portion 230 and the second portion 240 which are connected in sequence from top to bottom along a depth direction of the slot structure. A suspension structure may be provided on a sidewall of the first portion 230, and the first portion 230 may be suspended on the notch through the suspension structure, such that a clearance is formed between a bottom of the second portion 240 and an inner sidewall of the hollow hole. The second portion 240 may be located in the hollow hole, and a width at a widest position of the second portion 240 may be greater than a width of the notch.

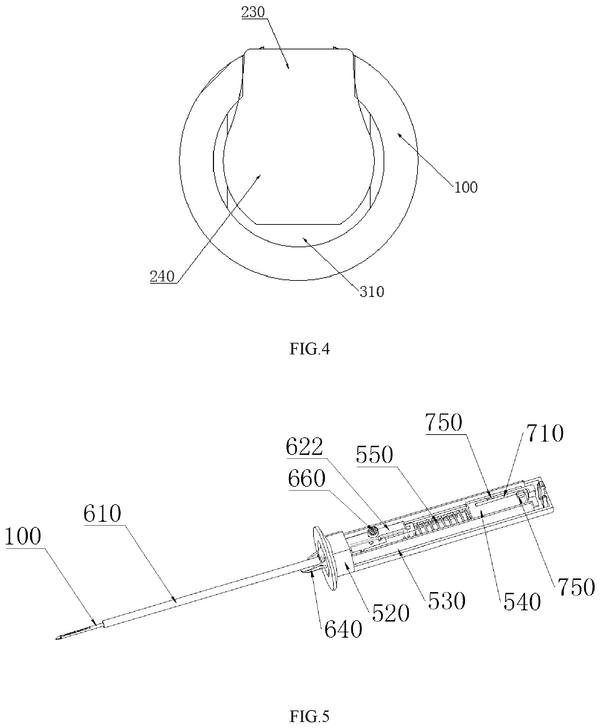

The suspension structure may be in various forms. For example, the implant may be a structure with a narrow upper portion and a wide lower portion, and a shape of a cross-section of the first portion 230 of the implant may be approximately rectangular; and the suspension structure may be provided at a position close to a top surface of the implant, the suspension structure may be a convex edge protruding to two sides, and the convex edge may be suspended outside the notch. As another example, the cross-section of the first portion may be an inverted trapezoid with a wide upper portion and a narrow lower portion, and a width at a widest position of the first portion may be greater than the width of the notch, such that the implant is suspended without falling and the bottom of the implant is prevented from contacting the bottom of the hollow hole. In some embodiments, the first portion 230 may be slidably connected to the notch of the puncture needle to prevent the implant from rotating and dislocating during a sliding process. In some embodiments, a shape of a cross-section of the second portion 240 may be an approximately incomplete spherical structure. In some embodiments, a bottom surface of the implant may be a plane. For example, the implant may be molded, and the bottom surface of the implant may be a plane during molding. In some embodiments, a bottom surface of the second portion 240 may be processed into a plane to increase a distance from a bottom surface of the circular hollow hole of the puncture needle, so as to prevent the puncture needle 100 from immediately contacting the bottom surface of the implant after the puncture needle 100 bends slightly to cause breakage of the implant. In some embodiments, the bottom surface of the second portion 240 may be in a shape that can form a deformation clearance (e.g., a deformation clearance 310 in FIG. 4) with the bottom surface of the puncture needle 100. The shape may include a regular shape or an irregular shape. For example, the bottom surface of the second portion 240 may be a concave curved surface, a serrated surface, an irregular shape matching the bone or tissue at the wound of the human body, etc., and the deformation clearance may be formed between the bottom surface of the second portion 240 and the bottom surface of the puncture needle. After the puncture needle 100 bends slightly, the deformation clearance can prevent the puncture needle 100 from immediately contacting the bottom surface of the implant, thereby preventing the implant from being squeezed by the puncture needle 100 and thus broken.

In some embodiments, the first chamfer 320 and the second chamfer 330 may be within a range of 8°-10°, respectively. For example, the first chamfer 320 and the second chamfer 330 may be 8°, 9°, or 10°, etc. An angle of the first chamfer 320 and the second chamfer 330 refers to an angle between a chamfered surface and an original circumferential sidewall or a surface of the implant. The larger the angle of the first chamfer and the second chamfer, the better the avoidance effect. If the angle is too large, the strength of the implant may decrease. When the first chamfer 320 and the second chamfer 330 are within the range of 8°-10°, the avoidance effect and the strength of the implant can both be considered. In some embodiments, the implant may be provided with a hole. The hole may be used as a suturing path for the suturing device; and a suture may pass through the hole.

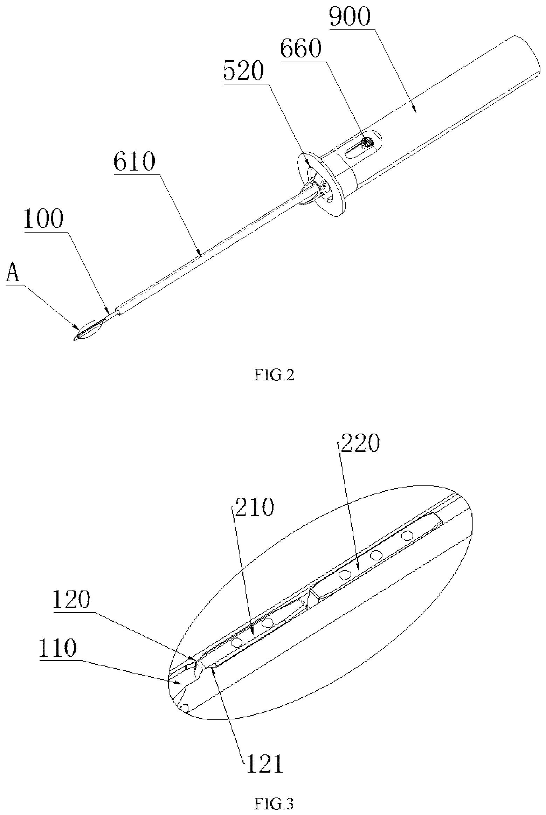

In some embodiments, as shown in FIG.1-12, the suturing device may include the puncture needle 100 and a plurality of implants. A count of implants may be two, three, or more. In some embodiments of the present disclosure, two implants may be taken as an example. In some embodiments, as shown in FIG. 2a, the plurality of implants are disposed on the puncture needle 100; the puncture needle 100 is provided with the channel 110 extending along an axial direction, the plurality of implants are arranged in the channel 110, and the plurality of implants are triggered from the suturing device after passing through the channel 110 in sequence. In some embodiments, the at least two limiting structures 120 may block the one or more implants. For example, the at least two limiting structures 120 may block all the implants. As another example, when a plurality of implants are provided, the at least two limiting structures 120 may block some of the implants. In some embodiments, the at least two limiting structures 120 are located on a triggering path of the implant. In some embodiments, the at least two limiting structures 120 are disposed on a triggering path from the second implant to be triggered to a front end of the puncture needle 100. For example, when two implants are arranged in the puncture needle, one of the two implants close to the front end of the puncture needle 100 is the first implant to be triggered, and the other of the two implants away from the front end of the puncture needle 100 is the second implant to be triggered. The at least two limiting structures 120 may be disposed at a position between the second implant to be triggered (hereinafter referred to as a second implant) and the first implant to be triggered (hereinafter referred to as a first implant). The at least two limiting structures 120 can at least block a second implant 220. Under the blocking effect of the at least two limiting structures 120, a friction force on the second implant 220 is increased, and it is more difficult for the second implant 220 to pass through the position where the at least two limiting structures 120 are located; the operator or the suturing device needs to apply a greater external force to the second implant 220 to make the second implant 220 pass through the at least two limiting structures 120, thereby reducing the risk of the first implant 210 bringing the second implant 220 out.

It should be noted that the at least two limiting structures 120 play a role in blocking rather than stopping. The second implant 220 can still pass through the at least two limiting structures 120, but it is more difficult to pass through the position in the channel 110 where the at least two limiting structures 120 are provided than the position in the channel 110 where no two limiting structure 120 is provided.

In some embodiments, a plurality of limiting structures 120 are provided in a circumferential direction of the puncture needle 100. In the circumferential direction, two or more limiting structures 120 are provided. When the implant passes through the position where the limiting structures 120 are located, the plurality of limiting structures are in sliding contact with the circumferential sidewall of the implant simultaneously to increase a friction force on the implant, thereby achieving a better blocking effect. In some embodiments, in the circumferential direction, an even count of limiting structures 120 may be provided and arranged opposite to each other in pairs. For example, two, four, or six limiting structures 120 may be provided, and half of the limiting structures 120 may be arranged on each of the two sides of the notch of the puncture needle 100. That is, one, two, or three limiting structures may be arranged on each of the two sides of the notch of the puncture needle 100.

In some embodiments, as shown in FIG. 3, each of the limiting structures 120 may include a guide slope 121 obliquely arranged relative to the inner wall of the channel 110. The implant may be in sliding contact with the guide slope 121. The guide slope 121 may be configured to guide the implant to cross over the limiting structures 120.

Merely by way of example, the limiting structures 120 may be wedge-shaped or hemispherical, and a smoothly transition may be formed between a rear edge of the guide slope 121 and the inner wall of the channel 110; from back to front, the guide slope 121 may gradually tilt toward a centerline of the channel 110, and the guide slope 121 may be a plane or a curved surface. The implant may move forward from a rear side of the limiting structures 120 and first contact the guide slope 121. Under the action of the guide slope 121, a pressure of the limiting structures 120 on the implant gradually increases, and the friction force on the implant gradually increases, such that a greater external force needs to be applied to the second implant 220 to make the second implant 220 pass through the limiting structures 120.

In some embodiments, the limiting structure 120 may block the one or more implants. For example, the limiting structures 12 may block all the implants. That is, all the implants, especially the first implant 210, may be blocked by the limiting structures 120, thereby preventing the first implant 210 from slipping out of the puncture needle 100. All the implants may have exactly the same shape, which makes the preparation of the implants more convenient and more versatile. In some embodiments, the first implant may be provided with an avoidance structure for avoiding the limiting structures 120, such that the friction force on the first implant when passing through the limiting structures may be less than the friction force on the second implant when passing through the limiting structures, and the first implant may pass through the limiting structures 120 more easily than the second implant. In some embodiments, the friction force on the first implant 210 caused by the limiting structures 120 is relatively small, such that the blocking effect on the first implant is relatively small. In some embodiments, the avoidance structure may include an avoidance slot. The avoidance slot may be disposed at a position of the first implant 210 opposite to the limiting structures 120. In some embodiments, the avoidance slot may connect a front end and a rear end of the first implant 210 along the axial direction. When the first implant 210 passes through the position where the limiting structures 120 are located, the limiting structures 120 may slide through the avoidance slot. In some embodiments, the avoidance structure may include that an outer diameter of the first implant 210 is less than an outer diameter of the second implant 220, thereby reducing a contact area between the limiting structures 120 and the first implant 210, reducing the friction force, and facilitating the triggering of the first implant.

In some embodiments, as shown in FIG. 3-FIG. 4, the puncture needle 100 may include a hollow hole, and a notch 111 communicated with the hollow hole may be provided on the circumferential sidewall of the puncture needle 100. In the axial direction, a front end of the notch 111 may be communicated with an end surface of a tip 114 of the puncture needle 100. The notch 111 and the hollow hole may enclose to form a slot structure 112. The slot structure 112 may form the channel 110. Each of the limiting structures 120 may include a protrusion located on an inner surface of the slot structure.

The implant may be located in the slot structure. When the implant slides along the slot structure and contacts the protrusions of the limiting structures 120, slot walls may drive the protrusions to press against the sidewall of the implant, thereby increasing the friction force and achieving the blocking effect on the second implant. Under the expansion of the implant, the two slot walls of the slot structure may slightly deform to increase a width of the notch of the slot structure, such that the second implant may pass through the limiting structures 120 without being blocked. The slot structure can more conveniently achieve the blocking effect on the second implant using the deformation of the slot structure, which makes the structure simpler.

In some embodiments, as shown in FIG. 4, protrusions 113 are disposed on two opposite sidewalls of the slot structure 112. The two protrusions 113 may be disposed opposite to each other, and the two protrusions 113 may be a hemispherical structure, respectively.

In some embodiments, as shown in FIG. 3-FIG. 4, the puncture needle 100 may include a hollow hole, and a notch communicated with the hollow hole may be provided on the circumferential sidewall of the puncture needle 100. In the axial direction, a front end of the notch may be communicated with an end surface of a tip of the puncture needle 100. The notch and the hollow hole may enclose to form a slot structure . The slot structure may form the channel 110. Each of the limiting structures 120 may include a protrusion located on an inner surface of the slot structure.

In general, the operator makes a slope of the tip 114 of the puncture needle 100 point upward and then inserts the puncture needle 100 downward for puncturing. During the puncturing, when the puncture needle 100 is blocked, the puncture needle 100 may bend slightly, and the bent channel 110 may gradually approaches the bottom surface of the implant. Since the clearance is reserved between the bottom surface of the implant and the bottom surface of the slot structure, the inner wall of the bent puncture needle 100 can be prevented from immediately contacting the implant to cause the implant to break. In some embodiments, the deformation clearance 310 may be within a range of 0.05 mm-0.4 mm. In some embodiments, the deformation clearance 310 may be within a range of 0.1 mm-0.2 mm.

In some embodiments, as shown in FIG. 5, the suturing device may include a fixing portion 400 and a triggering mechanism. The puncture needle 100 may be connected with the fixing portion 400. The triggering mechanism may move along an axial direction relative to the fixing portion 400. The triggering mechanism may be configured to trigger one or more implants toward a front end. In some embodiments, when the one or more implants are triggered, the puncture needle 100 and the fixing portion 400 may not move, and the triggering mechanism may push the one or more implants to move along the axial direction of the puncture needle 100, such that the one or more implants may move to the front end of the puncture needle 100.

In some embodiments, the suturing device may further include a housing 900. A portion of the fixing portion 400 may be located inside the housing 900 and fixedly connected to the housing 900. The puncture needle 100, the fixing portion 400, and the housing 900 may be relatively fixed.

In some embodiments, the triggering mechanism may push the one or more implants located on the puncture needle 100 forward through a push pin 510. In some embodiments, as shown in FIG. 5, FIG. 7 and FIG. 8, the triggering mechanism may include the push pin 510, a push button 520, a connection arm 530, and a linkage portion 540. The push button 520, the connection arm 530, and the linkage portion 540 may be arranged in sequence along a direction from a front end of the suturing device to a rear end of the suturing device. A front end of the push pin 510 may be located in the hollow hole of the puncture needle 100. The push button 520 may be slidably connected with the fixing portion 400. The push button 520 may move in a front-back direction. A front end of the connection arm 530 may be connected with the push button 520, and a rear end of the connection arm 530 may be connected with the linkage portion 540. The linkage portion 540 may be connected with a rear end of the push pin 510. In some embodiments, the push button 520, the connection arm 530, the linkage portion 540, and the push pin 510 may be fixedly connected and have the same movement state. In some embodiments, the connection arm 530 may connect the push button 520 and the linkage portion 540 as a whole, and the push pin 510 may be located inside the whole formed by connection of the connection arm 530, the push button 520, and the linkage portion 540. When any one of the push button 520, the connection arm 530, or the linkage portion 540 is pushed forward relative to the housing 900, a forward thrust may be transmitted to the linkage portion 540 and the push pin 510 to make the push pin 510 move forward. After the push button 520 is pushed forward relative to the housing 900, the linkage portion 540 may drive the push pin 510 to move forward, thereby pushing the implant in the channel 110 forward until pushing the implant out of the puncture needle 100. In some embodiments of the present disclosure, by driving the push pin to move through the connection arm 530 and the linkage portion 540, the push button can be disposed at a position convenient for the operator to control rather than necessarily being disposed at the rear end of the push pin, such that the structure of the suturing device is more in line with human mechanics. For example, when a person holds the suturing device with a hand, the push button is located at the position of the thumb, which is convenient for human operation.

In some embodiments, a spring 550 is provided between the fixing portion 400 and the linkage portion 540. When the push button 520 moves forward, the linkage portion 540 may squeeze the spring 550. When an external force on the push button 520 is released, the spring 550 may be configured to drive the linkage portion 540 to move in a direction away from the fixing portion 400, thereby resetting the push button 520. In some embodiments, two ends of the spring 550 may be fixed to the fixing portion 400 and the linkage portion 540, respectively.

In some embodiments, the suturing device may further include a depth limiting tube 610 and a locking mechanism. The depth limiting tube 610 may sleeve an outer side of the puncture needle 100, and a front end of the depth limiting tube 610 may be a depth limiting end. A position of the depth limiting tube 610 in the axial direction is adjustable. The locking mechanism may be configured to lock the depth limiting tube 610 such that the position of the depth limiting tube 610 may be fixed relative to the puncture needle 100. In some embodiments, the position of the depth limiting tube 610 in the axial direction being adjustable means that a distance between the depth limiting tube 610 and the front end of the puncture needle 100 can be adjusted.

In some embodiments, the depth limiting tube 610 sleeves the outer side of the puncture needle 100, and the front end of the depth limiting tube 610 may be a limiting end. By sliding the depth limiting tube 610, the distance between the depth limiting tube 610 and the front end of the puncture needle 100 can be changed, thereby changing a length of the puncture needle 100 exposed to the outside. Before suturing, a thickness of a tissue to be sutured may be measured first, and then the position of the depth limiting tube 610 may be adjusted and locked such that a length of a portion of the puncture needle 100 exposed at the front end of the depth limiting tube 610 matches the thickness of the tissue to be sutured. During the suturing process, the limiting end may abut against a surface of the tissue.

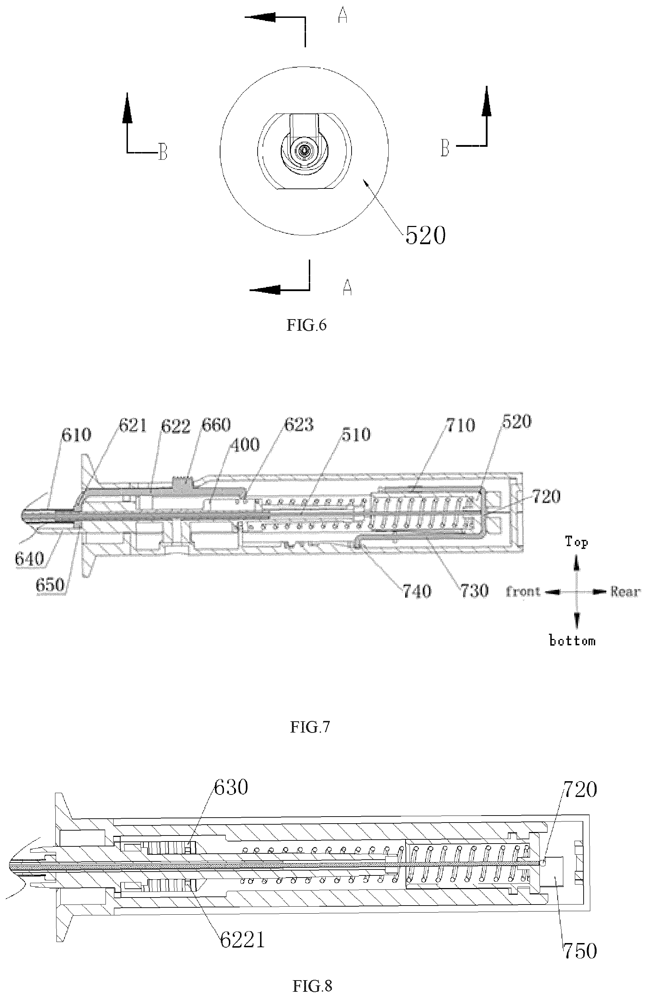

In some embodiments, as shown in FIG. 7 and FIG. 8, the locking mechanism may include a control arm. The control arm may include a first connection arm 621, a pressing arm 622, and a second connection arm 623 which are connected in sequence along the axial direction. In some embodiments, the first connection arm 621 and the second connection arm 623 may be arranged at an angle with the pressing arm 622, respectively, and the control arm may be approximately in an "inverted U" shape. One end (e.g., a lower end) of the first connection arm 621 may be fixedly connected with the depth limiting tube 610, and the other end (e.g., an upper end) of the first connection arm 621 may be connected with one end (e.g., a front end) of the pressing arm 622. One end (e.g., an upper end) of the second connection arm 623 may be connected with the other end (e.g., a rear end) of the pressing arm 622, and the other end (e.g., a lower end) of the second connection arm 623 may be slidably connected with the fixing portion 400. In some embodiments, the other end of the second connection arm 623 may abut against the fixing portion 400 and may slide along the axial direction relative to the fixing portion 400.

In some embodiments, a clearance is provided between the pressing arm 622 and the fixing portion 400 such that the pressing arm 622 is capable of moving relative to the fixing portion 400. In some embodiments, a gear pin 6221 is provided on the pressing arm 622. The locking mechanism may further include a plurality of limiting slots 630 located on the housing 900. The plurality of limiting slots 630 are arranged in sequence along the axial direction. The pressing arm 622 has a locked state and an unlocked state. In the locked state, the gear pin 6221 is located in one of the limiting slots 630, and the limiting slot 630 is configured to prevent the pressing arm 622 from moving along the axial direction. In the unlocked state, the gear pin 6221 is pressed out of the limiting slot 630, and the pressing arm 622 is capable of moving along the axial direction. In some embodiments, the operator may the pressing arm 622 toward the fixing portion 400 to cause the pressing arm 622 deform, the gear pin 6221 may be pressed out of the limiting slot 630, the pressing arm 622 may be switched to the unlocked state, and the pressing arm 622 is capable of moving along the axial direction. After the external force on the pressing arm 622 is released, the pressing arm 622 may return to an original state, and the gear pin 6221 may enter the limiting slot 630 corresponding to the position of the gear pin 6221 again. Through the cooperation between the gear pin 6221 and the limiting slots 630, a relative position of the depth limiting tube 610 and the puncture needle 100 can be conveniently adjusted.

In some embodiments, as shown in FIG. 7, a slide slot corresponding to the position of the pressing arm 622 may be provided on the housing 900. The slide slot connects the inside and outside of the housing 900. A button 660 may be connected to the pressing arm 622, and the button 660 may be configured to slide along the slide slot. By pressing the button 660 and pushing and pulling the button 660, the position of the depth limiting end can be changed and locked.

In some embodiments, a first guide slot 640 extending along the axial direction is provided on the fixing portion 400. A sliding abutting portion 650 is provided on the first connection arm 621. The sliding abutting portion 650 may abut against the first guide slot 640. The first guide slot 640 may be configured to guide the first connection arm 621 to slide along the axial direction of the puncture needle 100.

In some embodiments, after the pressing arm 622 is subjected to a pressure forcing the pressing arm 622 to deform toward the fixing portion 400, a bottom end of the second connection arm 623 may abut against the fixing portion 400. In some embodiments, the first guide slot 640 may be an outer edge of the fixing portion 400 extending toward the front end. The sliding abutting portion 650 may be a support foot provided at a bottom end of the first connection arm 621 extending downward. The support foot may abut against the outer edge. By providing the first guide slot 640, the first connection arm 621 can always move along the axial direction during a forward and backward sliding process without rotating; and the first connection arm 621 can be supported by the first guide slot 640.

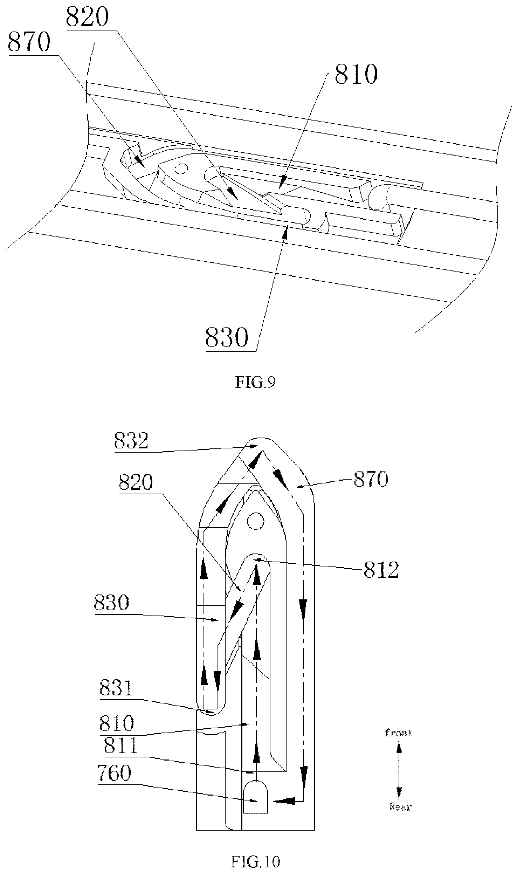

In some embodiments, as shown in FIGs. 9-12, the suturing device may include the housing 900 and a triggering mechanism. The triggering mechanism may be configured to trigger an implant toward a front end (i.e., in a direction close to the tip 114 of the puncture needle). A gear slot is provided on an inner wall of the housing 900. An elastic swing rod is provided on the triggering mechanism. A fixed end of the elastic swing rod may be fixedly connected with the triggering mechanism, and a free end 760 (see FIG. 10) of the elastic swing rod may extend from a notch of the gear slot into the gear slot. Merely by way of example, the gear slot may include a plurality of slots. When the free end 760 of the elastic swing rod extends into one of the slots, the triggering mechanism may be limited to a gear corresponding to the slot to control a position of the triggering mechanism in an axial direction.

In some embodiments, the triggering mechanism may be located on a side of the notch of the gear slot. The triggering mechanism may move in a front-back direction (e.g., a front-back direction shown in FIG. 7) along the axial direction. The elastic swing rod may move with the triggering mechanism. The elastic swing rod may include an elastic metal rod. One end of the elastic swing rod may be fixed to the triggering mechanism, and the other end of the elastic swing rod may be the free end 760. The free end 760 may extend into the gear slot. In some embodiments, during the movement of the triggering mechanism, the elastic swing rod may swing in a plane perpendicular to an opening of the gear slot, and may move toward or away from a bottom surface of the gear slot, such that the free end 760 may move along an extension path of the gear slot, thereby helping the operator to control a gear of the triggering mechanism. In some embodiments, a plane where the opening of the gear slot is located may be a plane parallel to B-B in FIG. 6; a plane perpendicular to the plane where the opening of the gear slot is located may be the plane shown in FIG. 7. That is, the elastic swing rod may swing up and down, and a swing direction of the elastic swing rod be forward approaching or backward away from the bottom surface of the gear slot.

In some embodiments, as shown in FIG. 7 and FIG. 8, the elastic swing rod is connected to the linkage portion 540. The elastic swing rod may include a first longitudinal rod 710, a first vertical rod 720, a second longitudinal rod 730, and a second vertical rod 740 which are connected end to end in sequence. The first longitudinal rod 710 and the second longitudinal rod 730 may be parallel to the axial direction. A rear end of the first longitudinal rod 710 may be connected with an upper end of the first vertical rod 720. A lower end of the first vertical rod 720 may be connected with a rear end of the second longitudinal rod 730. An upper end of the second vertical rod 740 may be connected with a front end of the second longitudinal rod 730. A lower end of the second vertical rod 740 may be the free end 760. The first vertical rod 720 may be located on a surface of the linkage portion 540 away from the gear slot. The first vertical rod 720 may be located on a rear end surface side of the linkage portion 540. The second vertical rod 740 may extend into the gear slot.In some embodiments, the connection between each rod and the linkage portion 540 may be achieved in various ways, such as snap connection, sleeve connection, etc. For example, the first longitudinal rod 710 and the first vertical rod 720 may be fixed to the linkage portion 540 through a limiting buckle 750. The limiting buckle 750 may be configured to prevent the first longitudinal rod 710 and the first vertical rod 720 from swinging relative to the linkage portion, respectively. In some embodiments, the limiting buckle 750 may also be provided on a surface of the linkage portion 540 facing the gear slot. The limiting buckle 750 may be close to the second longitudinal rod 730, and one end of the limiting buckle 750 away from the second vertical rod 740 may limit the second longitudinal rod 730. The second longitudinal rod 730 may swing with the limiting buckle 750 as a fulcrum. Merely by way of example, the limiting buckle 750 may be an arc slot arranged near an axial position of the puncture needle. The second longitudinal rod 730 may be a circular rod that is inserted into the arc slot. The circular rod may swing with the arc slot as a fulcrum, but the relative position of the second longitudinal rod 730 and the limiting buckle 750 in the axial direction remains constant. In some embodiments, an avoidance slot may be provided on the surface of the linkage portion 540 facing the gear slot to form a sufficient space for swinging above the second longitudinal rod 730. The second longitudinal rod 730 may swing in the plane perpendicular to the opening of the gear slot, or may approach the gear slot or move away from the gear slot in the direction of the opening of the gear slot.

In some embodiments, as shown in FIG. 9 and FIG. 10, two implants may be provided in the suturing device. The gear slot may include a first slot unit 810 and a second slot unit 830. The gear slot may also include a first retreat slot 820. A bottom surface of the first retreat slot 820 may be lower than a bottom surface of the first slot unit 810. One end of the first retreat slot 820 may be located on a bottom surface of a first limiting end 812 of the first slot unit, and may extend from the first limiting end 812 toward the rear end and pass through a side wall of the first slot unit 810 to communicate with the second slot unit 830. An extension direction of the first retreat slot 820 may be arranged at an angle to an extension direction of the first slot unit 810. A bottom surface of the second slot unit 830 may be lower than the bottom surface of the first retreat slot 820. The extension direction of the first retreat slot 820 being arranged at an angle to the extension direction of the first slot unit 810 means that the first retreat slot 820 may not parallel to the first slot unit 810, such that the free end 760 may be restricted by an end surface during movement. The bottom surface of the second slot unit 830 may be lower than the bottom surface of the first retreat slot 820. When the free end 760 retreats to an end of the first retreat slot 820, the free end 760 may directly enter an initial position of the second slot unit 830.

In some embodiments, at the initial position, the free end 760 may be located at a first starting end 811, and under an elastic force of the elastic swing rod, a lower end surface of the free end 760 may abut against the bottom surface of the first slot unit 810. The push button 520 may be pushed forward to drive the linkage portion 540 to move forward. The free end 760 may move along the first slot unit 810 to the first limiting end 812, the free end 760 may be blocked, and the operator knows that the first implant 210 is triggered. An end portion of the first retreat slot 820 may be located at the first limiting end 812, and the bottom surface of the first retreat slot 820 may be lower than the bottom surface of the first slot unit 810. That is, when the first implant 210 is triggered and the free end 760 moves to the first limiting end 812, the lower end surface of the free end 760 may perform a step-down movement under the elastic force of the elastic swing rod, and the free end 760 may enter the first retreat slot 820 and abut against the bottom surface of the first retreat slot 820. When the push button 520 is pushed backward, the free end 760 may be limited by a slot wall of the first retreat slot 820, and thus may move backward until moving to a connection between the first retreat slot 820 and the second slot unit 830. The free end 760 may perform another step-down movement, the free end 760 may enter the second slot unit 830, and the lower end surface of the free end 760 may abut against the bottom surface of the second slot unit 830. The push button 520 may be pushed forward again to make the free end 760 move to the second limiting end 832, thereby completing the triggering of the second implant 220.

In some embodiments, the gear slot may further include a second retreat slot 870. A bottom surface of the second retreat slot 870 may be lower than the bottom surface of the second slot unit 830. One end of the second retreat slot 870 may be located on a bottom surface of a second limiting end 832 of the second slot unit 830. The second retreat slot 870 may extend toward the rear end along the second limiting end 832 and may be connected with the first starting end 811 of the first slot unit 810.

In some embodiments, when the free end 760 moves to the second limiting end 832, the free end 760 may transition to the second retreat slot 870. When the operator pushes the push button 520 backward, the free end 760 may return to the first starting end 811 under the guidance of a slot wall of the second retreat slot 870. In the first slot unit 810, a bottom surface of the first starting end 811 may be lower than a bottom surface at a position between the first starting end 811 and the first limiting end 812. The bottom surface of the first starting end 811 of the first slot unit 810 may be an ascending slope, and a middle position of the first slot unit 810 may be a plane, so as to facilitate the free end 760 pushed out of the second retreat slot 870 to enter the first starting end 811 again.

In some embodiments, the ascending slope may be provided on the bottom surface of the second slot unit 830. The ascending slope may be located behind the second limiting end 832. The free end 760 may first perform an ascending movement and then fall into the second retreat slot 870. The ascending slope may facilitate the setting of the step-down structure at the transition of each slot.

In some embodiments, the free end 760 may be guided by the slot walls of the first slot unit 810, the first retreat slot 820, the second slot unit 830, and the second retreat slot 870 guide, so as to guide the free end 760 to move in each slot.

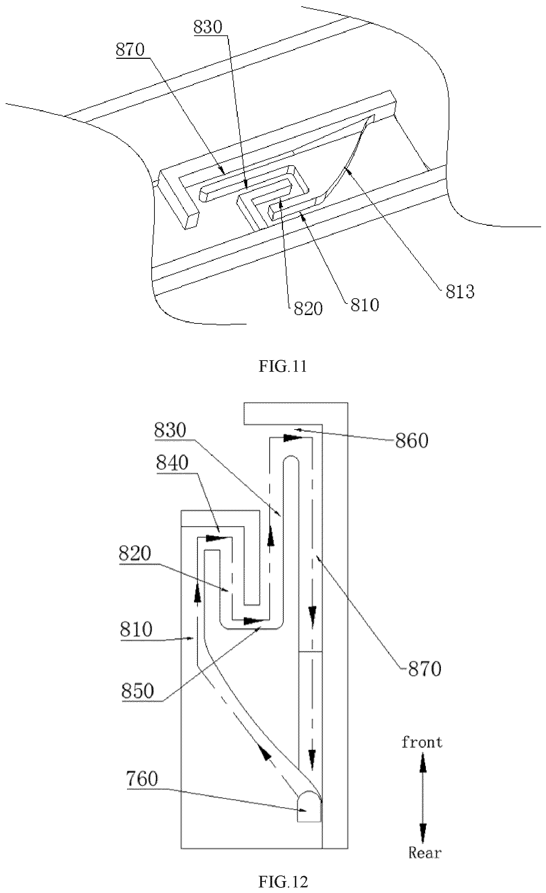

In some embodiments, as shown in FIG. 11 and FIG. 12, two implants may be provided in the suturing device. The gear slot may include the first slot unit 810 and the second slot unit 830. In some embodiments, the first slot unit 810 may include a first slot wall and a second slot wall 813. In some embodiments, as shown in FIG. 11 and FIG. 12, an extension direction of the first slot wall may be parallel to the axial direction, and a width between the second slot wall 813 and the first slot wall may gradually decrease in a direction from the rear end to the front end. In some embodiments, the second slot unit 830 may be located on a side of the second slot wall 813 of the first slot unit 810. The first retreat slot 820 may be provided between the first slot unit 810 and the second slot unit 830. A front end of the first retreat slot 820 may be connected with the first limiting end 812 of the first slot unit 810 through a first transverse slot 840, and a rear end of the first retreat slot 820 may be connected with the second starting end 831 of the second slot unit 830 through a second transverse slot 850. The second retreat slot 870 may be provided on the side of the second slot unit 830. The second slot unit 830 may be located between the first retreat slot 820 and the second retreat slot 870. A front end of the second retreat slot 870 may be connected with the second limiting end 832 of the second slot unit through a third transverse slot 860, and a rear end of the second retreat slot 870 may be connected with the second slot wall 813. A rear end bottom surface of the second retreat slot 870 may be higher than the bottom surface of the first starting end 811 of the first slot unit 810, so as to form a down-step structure at a connection point between the second retreat slot 870 and the first slot unit 810. In the initial state, the free end 760 may be located in the first slot unit 810 and located behind the opening of the rear end of the second retreat slot 870 and may abut against the second slot wall 813.

In some embodiments, in the initial state, the free end 760 may abut against the second slot wall 813 of the first slot unit 810. When the operator pushes the push button 520 forward, the free end 760 may move forward and always abut against the second slot wall 813, and the free end 760 may swing to store a part of elastic potential energy. When the free end 760 moves to the first limiting end 812, the free end 760 may swing into the first retreat slot 820 through the first transverse slot 840 under the driving of the elastic potential energy, and the free end 760 may abut against a slot wall in the first retreat slot 820 away from the first slot unit 810. The operator may push the push button 520 backward, and the free end 760 may move backward in the first retreat slot 820. When the free end 760 moves to the second transverse slot 850, the free end 760 may swing into the second slot unit 830 and abut against a sidewall of the second slot unit 830 away from the first slot unit 810 under the elastic action of the elastic swing rod. The push button 520 may be pushed forward, and the free end 760 may move to the second limiting end 832. The second implant 220 may be triggered. Meanwhile, the free end 760 may swing into the second retreat slot 870 through the third transverse slot 860. The push button 520 may be pushed backward, and the free end 760 may slide backward in the second retreat slot 870 and return to the first starting end 811.

In some embodiments, a material of the housing 900 may include a transparent material. The position of the free end 760 may be clearly seen through the housing 900, which is convenient for the operator to control the suturing device and better observe the progress of puncturing.

In some embodiments, the housing 900 may be formed by splicing an upper half housing and a lower half housing. The lower half housing and the fixing portion 400 may be fixedly connected by a threaded member.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure. Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure. Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. An implant, wherein a first chamfer is provided between a front end surface of the implant and a circumferential sidewall of the implant; and
a second chamfer is provided between a rear end surface of the implant and the circumferential sidewall of the implant.

2. The implant of claim 1, wherein a bottom surface of the implant is a plane.

3. The implant of claim 1, wherein the first chamfer (320) and the second chamfer (330) are within a range of 8°-10°, respectively.

4. A suturing device, comprising a puncture needle (100) and one or more implants, wherein a first chamfer (320 ) is provided between a front end surface of each of the one or more implants and a circumferential sidewall of each of the one or more implants, and a second chamfer (330 ) is provided between a rear end surface of each of the one or more implants and the circumferential sidewall of each of the one or more implants; the one or more implants are disposed on the puncture needle (100) ; the puncture needle (100) is provided with a channel (110 ) extending in an axial direction, and the one or more implants are capable of being triggered from the suturing device through the channel (110); and
at least two limiting structures (120) are disposed on an inner wall of the channel (110) and arranged along a circumferential direction; the at least two limiting structures (120 ) are capable of at least blocking a second implant to be triggered, such that an applied force is increased when the second implant to be triggered is triggered from the suturing device.

5. The suturing device of claim 4, wherein each of the at least two limiting structures (120 ) includes a guide slope (121 ) obliquely arranged relative to the inner wall of the channel (110 ), the one or more implants are in sliding contact with the guide slope (121 ), and the guide slope (121 ) is configured to guide the one or more implants to cross over the at least two limiting structures (120).

6. The suturing device of claim 4, wherein the at least two limiting structures (120 ) are capable of blocking the one or more implants.

7. The suturing device of claim 4, wherein an avoidance structure for avoiding the at least two limiting structures (120) is provided on a first implant (210) to be triggered, such that a friction force applied to the first implant (210) to be triggered when the first implant (210) to be triggered passes through the at least two limiting structures (120) is less than a friction force applied to the second implant (220 ) to be triggered when the second implant (220 ) to be triggered passes through the at least two limiting structures (120).

8. The suturing device of claim 4, wherein the puncture needle (100) includes a hollow hole, and a notch communicated with the hollow hole is provided on a circumferential sidewall of the puncture needle (100); in the axial direction, a front end of the notch is communicated with a tip end surface of the puncture needle, the notch and the hollow hole enclose to form a slot structure, and the slot structure forms the channel (110);
each of the at least two limiting structures (120 )s includes a protrusion located on an inner surface of the slot structure;

9. The suturing device of claim 8, two opposite sidewalls of the slot structure are provided with the protrusion, respectively, and the two protrusions are arranged opposite to each other.

10. The suturing device of claim 8 , wherein at least a portion of the one or more implants is located in the hollow hole, and when the portion of the one or more implants located in the hollow hole abuts against the notch of the slot structure, a deformation clearance (310 ) is formed between a bottom surface of the one or more implants and a bottom surface of the slot structure.

11. The suturing device of claim 10, the deformation clearance (310) is within a range of 0.05 mm-0.4 mm.

12. The suturing device of claim 10, wherein each of the one or more implants includes a first portion (230 ) and a second portion (240 ) which are connected from top to bottom along a depth direction of the slot structure;
a sidewall of the first portion (230 ) is provided with a suspension structure, and the first portion (230 ) is suspended on the notch through the suspension structure, such that a clearance is formed between a bottom of the second portion (240 ) and an inner sidewall of the hollow hole; and
the second portion (240 ) is located in the hollow hole, and a width at a widest position of the second portion (240 ) is greater than a width of the notch.

13. The suturing device of claim 4, further comprising a fixing portion (400 ) and a triggering mechanism, wherein the puncture needle (100)100 is connected with the fixing portion (400), the triggering mechanism is capable of moving along an axial direction relative to the fixing portion (400 ), and the triggering mechanism is configured to trigger the one or more implants toward a front end.

14. The suturing device of claim 13, wherein the triggering mechanism includes a push pin (510) , a push button (520) , a connection arm (530) , and a linkage portion (540) ; the push button (520) , the connection arm (530) , and the linkage portion (540) are arranged in sequence in a direction from a front end to a rear end of the suturing device;
a front end of the push pin (510) is located in the hollow hole of the puncture needle (100); and
the push button (520) is slidably connected with the fixing portion (400 ); a front end of the connection arm (530) is connected with the push button (520) , and a rear end of the connection arm (530) is connected with the linkage portion (540) ; the linkage portion (540) is connected with a rear end of the push pin (510) .

15. The suturing device of claim 14, wherein a spring (550) is provided between the fixing portion (400 ) and the linkage portion (540) .

16. The suturing device of claim 8, further comprising a depth limiting tube (610) and a locking mechanism, wherein the depth limiting tube (610) sleeves an outer side of the puncture needle (100) , a front end of the depth limiting tube (610) is a depth limiting end; a position of the depth limiting tube (610) in the axial direction is adjustable; and
the locking mechanism is configured to lock the depth limiting tube (610) such that the position of the depth limiting tube (610) is fixed relative to the puncture needle.

17. The suturing device of claim 16, wherein the locking mechanism includes a control arm, the control arm includes a first connection arm (621) , a pressing arm (622) , and a second connection arm (623) which are connected in sequence along the axial direction, one end of the first connection arm (621) is connected with the depth limiting tube (610) , and the other end of the first connection arm (621) is connected with one end of the pressing arm (622) ; one end of the second connection arm (623) is connected with the other end of the pressing arm (622) , and the other end of the second connection arm (623) is slidably connected with the fixing portion (400 );
a clearance is provided between the pressing arm (622) and the fixing portion (400) such that the pressing arm (622) is capable of moving relative to the fixing portion (400 ); a gear pin (6221) is provided on the pressing arm (622) ;
the locking mechanism further includes a plurality of limiting slots (630) s located on a housing (900) , and the plurality of limiting slots (630) s are arranged in sequence along the axial direction; and
the pressing arm (622) has a locked state and an unlocked state, in the locked state, the gear pin (6221) is located in one of the plurality of limiting slots (630) s, and the limiting slots (630) is configured to prevent the pressing arm (622) from moving along the axial direction, and in the unlocked state, the gear pin (6221) is pressed out of the limiting slot (630) , and the pressing arm (622) is capable of moving along the axial direction.

18. The suturing device of claim 17, wherein a first guide slot (640) extending along the axial direction is provided on the fixing portion (400), a sliding abutting portion (650) is provided on the first connection arm (621) , the sliding abutting portion (650) abuts against the first guide slot (640) , and the first guide slot (640) is configured to guide the first connection arm (621) to slide along the axial direction of the puncture needle.

19. The suturing device of claim 4, further comprising a housing (900) and a triggering mechanism, wherein the triggering mechanism is configured to trigger the one or more implants toward a front end; and
a gear slot is disposed on an inner wall of the housing (900) ; an elastic swing rod is disposed on the triggering mechanism, a fixed end of the elastic swing rod is fixedly connected with the triggering mechanism, and a free end (760) of the elastic swing rod extends into the gear slot from the notch of the gear slot.

20. The suturing device of claim 19, wherein a count of implants disposed in the suturing device are two, and the gear slot includes a first slot unit (810) and a second slot unit (830) ;
the gear slot further includes a first retreat slot (820) , a bottom surface of the first retreat slot (820) is lower than a bottom surface of the first slot unit (810) ; one end of the first retreat slot (820) is located on a bottom surface of a first limiting end (812) of the first slot unit (810) , and extends from the first limiting end (812) toward a rear end and passes through a sidewall of the first slot unit (810) to communicate with the second slot unit (830) , and an angle is formed between an extension direction of the first retreat slot (820) and an extension direction of the first slot unit (810) ; a bottom surface of the second slot unit (830) is lower than the bottom surface of the first retreat slot (820) ;
the gear slot further includes a second retreat slot, a bottom surface of the second retreat slot is lower than the bottom surface of the second slot unit (830) ; one end of the second retreat slot is located on a bottom surface of a second limiting end (832) of the second slot unit (830) , and extends from the second limiting end (832) toward the rear end and communicates with a first starting end (811) of the first slot unit (810) ; and
in an initial state, the free end (760) is located at the first starting end (811) , and the free end (760) has a tendency to move toward the bottom surface of the gear slot, such that the free end (760) always abuts against the bottom surface of the gear slot.

21. The suturing device of claim 19, wherein a count of implants disposed in the suturing device are two, and the gear slot includes the first slot unit (810) and the second slot unit (830) ;
the first slot unit (810) includes a first slot wall and a second slot wall (813) , an extension direction of the first slot wall is parallel to the axial direction; and a width between the second slot wall (813) and the first slot wall gradually decreases in a direction from the rear end to the front end;
the second slot unit (830) is located on a side where the second slot wall (813) of the first slot unit (810) is located;
the first retreat slot (820) is provided between the first slot unit (810) and the second slot unit (830) , a front end of the first retreat slot (820) is connected with the first limiting end (812) of the first slot unit (810) through a first transverse slot (840) , and a rear end of the first retreat slot (820) is connected with a second starting end (831) of the second slot unit (830) through a second transverse slot (850) ;
the second retreat slot (870) is provided on a side of the second slot unit (830) , and the second slot unit (830) is located between the first retreat slot (820) and the second retreat slot (870) ; a front end of the second retreat slot (870) is connected with the second limiting end (832) of the second slot unit (830) through a third transverse slot (860) , and a rear end of the second retreat slot (870) is connected with the second slot wall (813) , and a bottom surface of the rear end of the second retreat slot (870) is higher than a bottom surface of the first starting end (811) of the first slot unit (810) , such that a lower step structure is formed at a connection between the second retreat slot (870) and the first slot unit (810) ;
in the initial state, the free end (760) is located in the first slot unit (810) and behind an opening of the rear end of the second retreat slot (870) , and abuts against the second slot wall (813) .

22. The suturing device of claim 19, wherein a material of the housing (900) may include a transparent material.
